# EUROPEAN PATENT APPLICATION

(11) **EP 1 818 666 A1**
(43) Date of publication of application: **15.08.2007**
(21) Application number: 06101581.4
(22) Date of filing: 13.02.2006
(51) Int. Cl.: G01N 21/35, G01N 33/28

(54) **Determination of Sulphur in Marine Fuel Oils**

(71) Applicant: FOSS Analytical A/S, 3400 Hilleroed (DK)
(72) Inventor: Juhl, Henrik Vilstrup, DK-4000, Roskilde (DK)

(57) **Abstract**

An apparatus for the determination of sulphur content of marine fuel oil in a marine vessel propulsion system comprises a sampling station (8) adapted to present a sample of the marine fuel oil for analysis to an analyser having a complementary emitter (16,20) and detector (22,24,26) arrangement configured to respectively couple energy in one or both of the infra red and near infra red spectral regions into and to detect coupled energy after its interaction with, the marine fuel oil presented for analysis. The optical analyser further comprises a spectrometer (14) for generating a representation of an intensity variation of the detected coupled energy and computational means (28,32) adapted to analyse the representation to produce output signals (30) indicating a sulphur content of the marine fuel oil usable in control of functional elements of the marine vessel propulsion system.

## Description

The present invention relates to an apparatus for determining the presence of sulphur in fuel oils and in particular to the determination of sulphur in marine fuel oil of a marine vessel.

It is known from , for example, US 6859517 B (WILSON ET AL), to provide an apparatus for the determination of contamination in lubricants and in fuel oils on board a marine vessel. The apparatus employs an X-ray fluorescence spectrometer in the analysis of the fluids, including for the determination of the sulphur content of the marine fuel oil. One disadvantage with this system is that the X-ray source represents a limited bio-hazard to a user.

It is an aim of the present invention to provide an apparatus for the monitoring the sulphur content of marine fuel oil in-line, at-line or off-line in a marine vessel propulsion system that at least mitigates the disadvantage of the known apparatus.

According to the present invention there is provided an apparatus for the determination of the sulphur content of marine fuel oil as described in and characterised by the present Claim 1. By employing a suitably calibrated spectrometer which is designed to operate in one or both the infra red and near infra red wavelength regions, the sulphur content may be determined periodically without recourse to the use of X-ray sources.

Usefully, the so determined sulphur content may be used to control the operation of functional elements of the marine propulsion system.

Most advantageously control of the amount of lubricant supplied to combustion cylinders of the engine of the marine vessel propulsion system may be made dependent on the so determined sulphur content.

These and other advantages will be made apparent from a consideration of the description below of embodiments of the apparatus according to the present invention and made with reference to the drawings of the accompanying figures, of which:

Fig. 1 illustrates schematically an embodiment of the apparatus according to the present invention including portions of a marine propulsion system;

Fig. 2 illustrates schematically a further embodiment according to the present invention; and

Fig. 3 illustrates schematically elements of a lubricant supply means of a marine vessel propulsion system controllable according to the present invention.

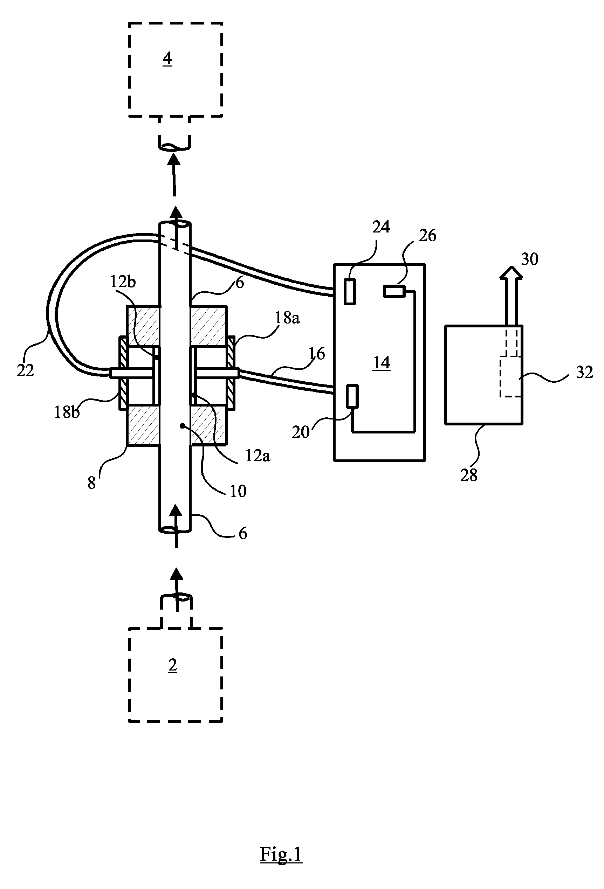

Considering now Fig.1, functional elements of a marine vessel propulsion system are illustrated as including a fuel tank 2 which is fluidly connected to a marine engine 4 by means of a fuel pipe 6 such that marine fuel oil can flow, generally in a direction indicated by the arrows in the figure, from the tank 2 to the engine 4 for combustion. A fuel pump (not shown) will be normally included in the marine vessel propulsion system in order to effect this flow.

As an element of the apparatus according to the present invention there is provided a sampling station 8, which in the present example is realised as a coupling piece located in-line with the fuel pipe 6. The sampling station 8 is, in the present example, generally cylindrical and conforms approximately to the outer circumferential dimensions of the fuel pipe 6. The sampling station 8 is provided with a through bore 10 such that, when located in-line, opposing open ends of the bore 10 establish a fluid connection with the fuel pipe 6 so that fuel can continue to flow essentially uninterrupted between the tank 2 and the engine 4 of the marine propulsion system. Some or all of the walls defining the bore 10 in the present embodiment are made of suitably transparent material in order to allow energy in one or both of the near infrared or infra red wavelength regions, to be directed into the fuel oil flowing in the pipe line 6 and to be collected after its interaction therewith. In the present example the transparent material is provided as a pair of windows 12a,b sited in opposition to one another along an axis substantially perpendicular to the direction of flow of the marine fuel oil through the sample station 8. It is to be understood that these windows 12a,b may be relatively orientated at other angles depending on, for example, the type of analysis to be performed (transmission, reflectance or trans-flectance measurements for example), design considerations or user accessibility or serviceability. Indeed, in some configurations only a single window may be necessary.

According to the exemplary embodiment depicted in Fig. 1 a spectrometer 14 is provided and functions in a known manner to generate an electrical representation of an intensity variation, resolved in either a wavelength or a frequency (time) domain, of the directed radiation after its interaction with fuel oil in the through bore 10 of the sampling station 8. As one example, the spectrometer 14 may be configured as a monochromator to provide the representations in the wavelength domain. By way of example, polychromatic radiation from an emitter 20, that is collocated with the spectrometer 14, may be transmitted along a fiber optic 16 that terminates proximal a one of the windows 12a and that is here held in place by a "light-tight", optionally removable, sealing element 18a. A second fiber optic 22 is provided with one end proximal the other window 12b to receive the polychromatic radiation after its interaction with the fuel oil flowing through the bore 10 of the sampling station 8 and to conduct it to the spectrometer 14. This fiber optic 22 may also be held in place at the sampling station 8 by means of a similar light tight sealing element 18b.

A dispersion element, such as a fixed diffraction grating 24 of the spectrometer 14 according to the present embodiment, is disposed to disperse the so conducted radiation on to a detector, here a position sensitive diode array 26. Each individual diode of the diode array 26 is adapted to generate an electrical signal having an intensity which is proportional to incident radiation.

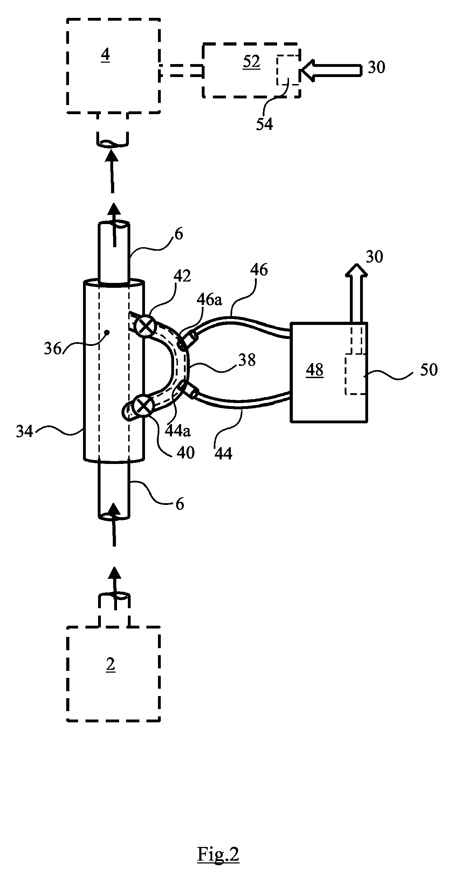

As is known, the wavelength of radiation from the dispersion element 24 that is incident on a diode of such an array 26 will be dependent on the position of that diode in the array 26. The electrical representation of the wavelength dependent intensity variations that is thus generated by the diode array 26 is passed to a computer, here a separate unit 28, that includes the necessary processing electronics and programming by which the electrical representation is processed to produce output signals 30 indicating a sulphur content of the marine fuel oil flowing in the fuel pipe 6. These signals 30 may then be employed in the control of the operation of elements of the marine vessel propulsion system, for example as will be described below with respect to Fig. 2.

The unit 28 may be in the form of a suitable commercially available general purpose personal computer and may include an appropriate user interface 32, such as a keyboard by which instructions are passed to the spectrometer 14 as well as a display by which at least a portion of the output signals 30 may be represented in a human readable form.

The programming employed to process the representation of the spectrum will normally implement a calibration model or algorithm that correlates spectral features from that spectrum obtained from the analysed sample of marine fuel oil with sulphur content. It will be appreciated that only certain spectral bands within the entire spectrum may be associated with the presence of sulphur so that a calibration model may be constructed that relies on some or all of these spectral bands. Consequently the spectrometer 14 may be configured so that only those electrical signals that represent intensities from those same spectral bands of interest are employed within the computer 28 in the determination of the sulphur content.

The science of chemometrics may be applied to the problem in a generally known manner whereby multivariate statistical analysis is employed in order to produce the calibration algorithm which establishes the correlation of the spectral signals with the concentration of sulphur. This involves the use of a set of a plurality of "training" or calibration samples which are preferably selected to span the range of sulphur concentrations and fuel oil compositional variations likely to be of interest. From the spectra obtained from these training samples and knowledge of the sulphur content in each sample a calibration equation or algorithm is established that links the spectroscopic data obtained from an analysis of a fuel sample and the quantified presence of sulphur. In this manner an indication of sulphur content of a sample may be determined that is insensitive to other variations in the chemical composition of the fuel oil itself.

It will be appreciated that the functional elements of the spectrometer 14 may be configured in a number of different ways whilst still achieving the functionality of a monochromator, for example the optical emitter 20 may be directly located proximal the window 12a and an electrical connection between the spectrometer 14 and the emitter 20 may be established in order to provide control of the energisation of the emitter 20. The dispersion element 24 need not be a fixed grating but instead be a one that is movable to effect dispersion. The detector 26 need not be position sensitive since the wavelength of energy incident on its sensitive surface is now dependent of the position of the moving grating 24.

It will also be appreciated that the computer 28 may be provided integral with the spectrometer 14 and may comprise a number of special purpose computational units which are designed specifically to accomplish one or more of the tasks to implement the present invention, such as may include data collection, correction and processing tasks to generate the necessary sulphur content information.

Consider now the exemplary embodiment of an apparatus according to the present invention which is illustrated in Fig. 2. A sampling station 34 is illustrated as having a through bore 36 through which in use marine fuel oil in the fuel pipe 6 will flow. This through bore 36 provides a main flow passage for the majority of the marine fuel oil that flows between a fuel tank and a marine engine (not shown) of a marine vessel propulsion system. Fluidly coupled to this through bore 36 is a by-pass conduit 38. Flow control means 40, 42, such as simple on/off valves, is also provided in the present embodiment to control the flow of marine fuel oil from the through bore 36, through the by-pass conduit 38 and back to the main flow, here in the through bore 36. Alternatively, the by-pass conduit 38 may be configured to permit the sample to be discarded or returned to the fuel tank 2 after analysis. This flow control means 40, 42 may usefully be operated to provide a flow of fuel oil in the by-pass conduit 38 only during analysis and in certain circumstances by be operated to stop the flow through the by-pass conduit 38 in order to perform an analysis on a stationary fuel oil sample that is here subsequently returned to the main flow through the fuel pipe 6.

Probes 44, 46 are provided as elements of the apparatus and here are each provided with respective ends 44a, 46a that terminate internal the by-pass conduit 38 and are relatively angled to facilitate the collection of that radiation which is emitted in one or both the infra red and near infra red spectral regions from a one of the probes (44 say) by the other of the probes (46 say) after passing through marine fuel oil in the by-pass conduit 38.

A spectrometer 48 is provided for operable connection to the probes 44, 46, for example by using optical fibers. This spectrometer 48 is configured to operate in a generally known manner, for example, as a monochromator or as a Fourier Transform spectrophotometer, in order to generate a representation of an intensity variation of the collected radiation in a form suitable for processing by a computer means 50 generally in a manner described in connection with the computer 28 of the embodiment illustrated in Fig. 1.

Thus the computer means 50, which in the present embodiment is provided integral with the spectrometer 48, is adapted to process the representation, typically by the application of a suitable calibration model that relates spectral characteristics to sulphur content of the analysed sample, to provide output 30 indicating a sulphur content of the marine fuel oil. In common with the embodiment of Fig. 1, this output 30 may then be employed in the control of the operation of one or more elements of the marine propulsion system.

For example, it is known that the marine engine 4 of a marine vessel propulsion system is not only supplied with fuel, here via the fuel pipe 6, but is also provided periodically with lubricant directly into the combustion chambers. The lubricant is typically sprayed or injected directly into the chambers in a controlled manner by a lubricant supply means 52. The amount supplied is generally proportional to an average sulphur content value that is expected of the entire fuel feedstock held in the fuel tank 2 of the propulsion system. This value may be provided by the fuel supplier and then entered manually into the control system of the lubricant supply means 52. More usually, this value may be factory set to a level intended to cater to a maximum expected sulphur content. A problem is that the sulphur content of the fuel feedstock may vary so that in the known propulsion system the lubricant may be supplied to the engine 4 in amounts more or less than the optimum amount for the sulphur content of the fuel to be combusted. According to the present invention the lubricant supply means 52 is adapted to receive periodic output 30 from the computational means 50 as the indication of the sulphur content that is to be employed in controlling the amount of lubricant being supplied to the engine 4. According to the embodiment of Fig. 2, the lubricant supply means 52 is illustrated schematically to include a pump 54 which here operates for a predetermined time to drive lubricant from a reservoir (not shown) and into the engine 4. The pump 54 is adapted to receive the output signal 30 as a timing signal to control the predetermined time the pump is operated dependent on the sulphur content. In this manner the amount of lubricant supplied to the engine 4 by the supply means 52 may be controlled in dependence of the sulphur content of the marine fuel oil intended for combustion. The value of the predetermined operational time may usefully be updated a plurality of times as the fuel tank 2 is emptied through use of the fuel oil. Consequently, the marine vessel propulsion system may be operated with increased efficiency to provide a saving in the use of one or both the fuel oil and the lubricant.

The foregoing description of the operation and the control of the lubricant supply means 52 is provided by way of example only and without limitation and is done so in order to permit the skilled person to gain a better understanding of the invention as claimed.

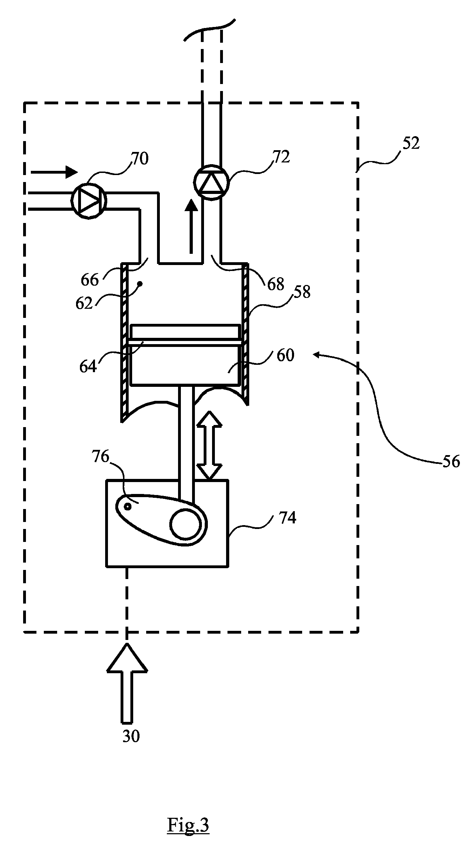

A further example of the lubricant supply means 52 is illustrated by Fig. 3. Here the pump 54 of Fig. 2 is replaced by a 'syringe-type' injector 56. The injector 56 comprises a generally cylindrical bore 58 within which a piston 60 is reciprocally movable (as indicated by the arrow) to vary a volume of a lubricant holding space 62. The piston 60 is provided with a circumferential seal 64 that acts in cooperation with the inner walls of the bore 58 to prevent seepage of lubricant past the piston 60. The bore 58 is provided with openings 66, 68 located towards the limit of travel of the piston 60 in the direction tending to minimize the volume of the lubricant holding space 62. One way valves 70, 72 are provided, a one of which being association with a respective opening 66, 68, and respectively arranged to ensure that lubricant from a reservoir (not shown) can flow into the lubricant holding space 62 only via the opening 66 and that lubricant in the lubricant holding space 62 can flow out of the space 62 to the engine (not shown) only via the opening 68 as the piston 60 reciprocates under control of a drive means 74. This drive means 74 may for example comprise a rotary motor provided with a cam surface 76 to which the piston 60 is mechanically connected such that together rotational motion of the motor is converted to reciprocal motion of the piston 60.

According to the present invention the drive means 74 is adapted to receive the output signals 30 and to operate in dependence thereon to limit the stroke length of the piston 30. For example, the movement of the piston 60 to cause an increase in the volume of the lubricant holding space 62 (so-called "expansion stroke") may be limited. The amount of lubricant drawn through the opening 66 during this expansion stroke is then dependent on the measured sulphur content of the marine fuel. This entire amount may then be injected into the engine as the piston 60 is moved to minimise the volume of the lubricant holding space 62 (so-called "compression stroke") and force lubricant to exit through the opening 68. Alternatively or additionally, the extent of the compression stroke may be limited such that the volume of the lubricant holding space 62 is not minimised but rather only reduced from that extant at the end of the expansion stroke of by an amount dependent the signal 30 indicating the determined sulphur content of the fuel.

In this manner the amount of lubricant passing through the opening 68 for supply to the engine is made dependent on the sulphur content of the fuel as measured by the spectrometer (48 of Fig. 2 say) of the apparatus according to the present invention.

It will be appreciated that whilst the sampling station 8, 34 is shown for each embodiment as a unit connectable in-line with a fuel pipe 6 of the marine vessel propulsion system the sampling unit may be adapted without departing from the invention as claimed, for example to directly sample marine fuel oil in the fuel tank 2 or it may be a cuvette, such as a manually fillable cuvette, coupled to the spectrometer for analysis in a known manner.

## Claims

1. An apparatus for the determination of sulphur content of marine fuel oil in a marine vessel propulsion system (2,4,6,52) **characterised in that** the apparatus comprises a sampling station (8;34) adapted to present a sample of the marine fuel oil for analysis; and an analyser having a complementary emitter (16,20;44) and detector (22,24,26;46) arrangement configured to respectively couple energy in one or both of the infra red and near infra red spectral regions into and to detect coupled energy after its interaction with, the marine fuel oil presented for analysis; the analyser further comprising a spectrometer (14;48) adapted to generate a representation of an intensity variation of the detected coupled energy and computational means (28;50) adapted to analyse the representation to determine a sulphur content of the marine fuel oil and to produce output signals (30) indicative of the same.

2. An apparatus as claimed in Claim 1 **characterised in that** the sampling station (8;34) is provided with a through bore (10;36) adapted for in-line coupling with a fuel carrying pipe (6) of the marine vessel propulsion system (2,4,6,52).

3. An apparatus as claimed in Claim 1 or Claim 2 **characterised in that** the sampling station (34) is configured with a by-pass conduit (38) to present the sample of the marine fuel oil for analysis by diverting marine fuel oil from that flowing in the fuel pipe (6).

4. An apparatus as claimed in any of the preceding claims **characterised in that** at least a portion of the output signal (30) is constituted as a control signal for controlling the operation of the marine vessel propulsion system (2,4,6,52).

5. An apparatus as claimed in Claim 4 **characterised in that** the at least a portion of the output signal (30) is constituted as a control signal for controlling the operation of a suitably adapted lubricant supply means (52) to supply lubricant to an engine of the marine vessel propulsion system (2,4,6,52) dependent on the determined sulphur content.

6. An apparatus as claimed in any preceding claim **characterised in that** the computational means (28;50) is configured to operate to apply a calibration algorithm which establishes a correlation of the representation to the concentration of sulphur; said calibration algorithm being derived by multivariate analysis of a plurality of training samples of known sulphur concentration selected to span a range of sulphur concentrations and fuel oil compositional variations likely to be of interest.
